(19) Europäisches Patentamt
European Patent Office
Office européen des brevets

(11) **EP 4 464 166 A1**

(12) **EUROPEAN PATENT APPLICATION**

(43) Date of publication:
**20.11.2024 Bulletin 2024/47**

(21) Application number: **23188907.2**

(22) Date of filing: **01.08.2023**

(51) International Patent Classification (IPC):
**A23K 10/20** (2016.01)     **A01K 61/00** (2017.01)
**A23K 10/30** (2016.01)     **A23K 20/174** (2016.01)
**A23K 20/20** (2016.01)     **A23K 50/80** (2016.01)

(52) Cooperative Patent Classification (CPC):
**A23K 50/80; A23K 10/20; A23K 10/30;
A23K 20/174; A23K 20/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **15.05.2023   CN 202310544510**

(71) Applicant: **Petidea Capital Investment Limited
Kowloon Bay Kowloon (HK)**

(72) Inventor: **WONG, Chi-Kin
Kowloon Bay - Kowloon (HK)**

(74) Representative: **Bureau M.F.J. Bockstael NV
Tavernierkaai 2
2000 Antwerp (BE)**

(54) **TREATMENT AGENT AND METHOD FOR IMPROVING ATTRACTIVENESS OF INSECTS OR THEIR LARVAE TO AQUACULTURE ANIMALS**

(57)     Disclosed is a treatment agent and a method for improving attractiveness of insects or their larvae to aquaculture animals. The treatment agent described in the present disclosure comprises at least one selected from the group consisting of nitrate and nitrite, and at least one selected from the group consisting of ascorbic acid, isoascorbic acid, tea polyphenols and nicotinamide. The treatment method comprises the following steps: washing the insects or their larvae; soaking the washed insects or their larvae in a solution of the treatment agent described in the present disclosure to obtained soaked insects or their larvae; and taking the soaked insects or their larvae out for draining water to obtain treated insects or their larvae. The treatment method of the present disclosure can kill pathogens in insects and fix the hemoglobin of insects, and improve the attractiveness and palatability of the obtained insects or their larvae.

EP 4 464 166 A1

Figure 1

## Description

### TECHNICAL FIELD

The present disclosure belongs to the field of animal feed technology, and specifically relates to a treatment agent and a method for improving attractiveness of insects or their larvae to aquaculture animals.

### BACKGROUND

Insects are rich in protein and are known for their minimal cultivation. Insects as a kind of food are rich in protein, low fat content and many vitamins and other nutrients, and insect protein as a kind of new protein resource has a rich variety and sustainable development. For omnivorous and carnivorous freshwater fish, insects are a part of their daily diet. Insect protein can provide nutritional value and odor different from those of fish meal, and is a high-quality protein source for aquaculture animals.

After harvesting, insects are traditionally washed with water and then preserved in different ways. In the related technology, the common ways of preserving insects mainly include ozone or hypochlorous acid disinfection, sterilization by adding sterilizing agents, drying, freezing or fermentation, etc. Chinese patent CN109965154 A describes a preservation process of animal edible insect, in which insects are preserved by freezing after sterilization with ozone water, high temperature and microwave. Chinese patent CN109691615 A describes a low-temperature quick micro-freezing fresh-keeping technology for edible specimens and breed insects, in which insects are frozen directly in microfreezing liquid to preserve freshness. Chinese patent CN 113273639 A describes a preparation method of fresh-keeping insects containing probiotic, in which pasteurization method and mixed probiotic fermentation solution are used to inhibit the growth of spoilage microorganisms for the purpose of preserving insects.

The above-mentioned insect treatment methods have their own advantages and disadvantages. Although sterilizing agents are less expensive, most of sterilizing agents cannot completely remove the sediment inside the intestinal tract because they only act on the insect body surface. Ozone and hypochlorous acid disinfection will cause the insect body to turn black and the protein and flavor amino acid to deteriorate, which affects the attractiveness to aquaculture animals. Although drying can reduce transportation costs and prolong storage time, high temperature will easily lead to nutrient loss and oxidation, which can cause the product to turn black and affect the quality and appearance, and also reduce the attractiveness and palatability of insects. Freezing can maintain nutrients and active substances, but direct freezing without sterilization can easily retain pathogens in the insects, thus affecting the health of aquaculture animals and causing uncontrollable economic losses. Although the acidic fermentation broth of probiotics can effectively prevent the growth of spoilage microorganisms, the insects still need to be supplemented with other sterilization methods to make the insects meet the required hygienic standards. The high-temperature sterilization will inevitably affect the appearance and flavor of insects.

Therefore, there is a need for a treatment technology that can effectively kill the pathogens in the insects and clean the sediment inside the intestinal tract, and ensure the original quality and appearance of the insects, as well as give full play to the advantages of the insects in the feeding of aquaculture animals in terms of attractiveness and palatability.

### SUMMARY

The present disclosure aims to solve at least one of the technical problems of the prior art as described above. To this end, in a first aspect of the present disclosure, provided is a treatment agent of insects or their larvae that can effectively kill pathogens in the insects or their larvae and ensure the original quality and appearance of the insects or their larvae.

In some embodiments of the present disclosure, the treatment agent comprises at least one selected from the group consisting of nitrate and nitrite, and at least one selected from the group consisting of ascorbic acid, isoascorbic acid, tea polyphenols, and nicotinamide.

In the present disclosure, alternatively, the ascorbic acid, isoascorbic acid, tea polyphenols and nicotinamide may be ethoxy quinoline, butylhydroxyanisole (BHA), dibutylhydroxytoluene (BHT), propyl gallate, tertiary butylhydroquinone, vitamin E, L-ascorbyl-6-palmitate, rosemary extract, dilauryl thiodipropionate, licorice antioxidant, phytic acid (inositol hexaphosphate) and other antioxidants.

In some embodiments of the present disclosure, the treatment agent further comprises water.

In some embodiments of the present disclosure, the treatment agent comprises nitrate, nitrite, ascorbic acid and water.

In some embodiments of the present disclosure, the treatment agent comprises nitrite, isoascorbic acid and water.

In some embodiments of the present disclosure, the treatment agent comprises nitrite, ascorbic acid and water.

In some embodiments of the present disclosure, the treatment agent comprises nitrite, tea polyphenols and water.

In some embodiments of the present disclosure, the treatment agent comprises nitrite, nicotinamide and water

In some embodiments of the present disclosure, the treatment agent comprises 4 to 20% by weight of nitrate and/or 4 to 20% by weight of nitrite, and 10 to 40% by weight of ascorbic acid, isoascorbic acid, tea polyphenols or nicotinamide.

In some embodiments of the present disclosure, the treatment agent further comprises water, wherein the amounts of nitrate and/or nitrite, and ascorbic acid, isoascorbic acid, tea polyphenols or nicotinamide are combined to make 100%.

In some embodiments of the present disclosure, the treatment agent comprises 5 to 20% by weight of nitrate and/or 4 to 15% by weight of nitrite, and 20 to 40% by weight of ascorbic acid, 25 to 40% by weight of isoascorbic acid, 10 to 20% by weight of tea polyphenols or 15 to 30% by weight of nicotinamide, with a balance being water.

In some embodiments of the present disclosure, the nitrate is at least one selected from the group consisting of sodium nitrate, potassium nitrate, and the nitrite is at least one selected from the group consisting of sodium nitrite, potassium nitrite.

In some embodiments of the present disclosure, the insects or their larvae are insects or their larvae containing hemoglobin; the insects or their larvae containing hemoglobin include *Chironmidae larvae*, *Lumbricus terrestris*, *Tubifex*, *Muscomorpha*, and *Nereis succinea.*

In a second aspect of the disclosure, provided is a method of treating insects or their larvae, comprising the steps of:

S1. washing the insects or their larvae to obtain washed insects or their larvae; and

S2. soaking the washed insects or their larvae in a solution of treatment agent described in the first aspect of the present disclosure to obtained soaked insects or their larvae; and taking the soaked insects or their larvae out for draining water to obtain treated insects or their larvae.

In some embodiments of the present disclosure, in Step S2, soaking the washed insects or their larvae in the solution of treatment agent for 0.5 to 16 hours.

In some embodiments of the present disclosure, the treatment method further comprises: immersing the insects or their larvae in a nutrient solution after soaking in the treatment agent solution; and the immersing lasts for 0.5 to 3 hours.

In some embodiments of the present disclosure, the nutrient solution in the step S3 comprises 5~15% by weight of complex vitamins and 0.5~3% by weight of complex trace elements.

In some embodiments of the present disclosure, the nutrient solution comprises complex vitamins and complex trace elements. The complex vitamins comprises one or more selected from the group consisting of vitamin $B_1$, vitamin $B_2$, vitamin $B_3$, vitamin $B_{12}$, vitamin C, vitamin D, and vitamin E. The complex trace elements comprises one or more selected from the group consisting of calcium, magnesium, phosphorus, zinc, manganese, copper, iron, and selenium.

In some embodiments of the present disclosure, the multivitamin comprises 0.1~0.5% by weight of vitamin $B_1$, 0.1~0.5% by weight of vitamin $B_2$, 0.4~0.8% by weight of vitamin $B_3$, 0.01~0.10% by weight of vitamin $B_{12}$, 2~10% by weight of vitamin C, 1~3% by weight of vitamin D, 5~10% by weight of vitamin E; and the trace element complex comprises 1~3% by weight of calcium, 0.01~0.03% by weight of iodine, 0.5~2% by weight of zinc, 0.05~0.3% by weight of copper, 0.001~0.003% by weight of cobalt, 0.00001~0.00002% by weight of selenium.

In some embodiments of the present disclosure, the complex vitamins and complex trace elements also comprises water, wherein the amount of water makes the total amount of complex vitamins or the total amount of complex trace elements to 100%.

In some embodiments of the present disclosure, the immersing in the Step S3 lasts for 0.5 to 3 hours.

In some embodiments of the present disclosure, a mass ratio of insects, treatment agent solution and nutrient solution is (10-30): (0.5-4): (1-4).

In some embodiments of the present disclosure, the mass ratio of insects, treatment agent solution and nutrient solution is (10-20): 1: (1-2).

In some embodiments of the present disclosure, the insects or their larvae obtained by the treatment method may be subsequently further processed, including but not limited to, cryogenic freezing, drying, lyophilization, fermentation.

In some embodiments of the present disclosure, a temperature of the cryogenic freezing is -20°C to -40°C.

In some embodiments of the present disclosure, the cryogenic freezing lasts for 20 minutes to 40 minutes.

In some embodiments of the present disclosure, the lyophilization is operated at a vacuum condition.

In some embodiments of the present disclosure, a temperature of the lyophilization is -20°C to - 30°C.

In some embodiments of the present disclosure, the lyophilization lasts for 15 hours to 25 hours.

In a third aspect of the present disclosure, provided is use of insects or their larvae obtained by the treatment method described in the second aspect in the preparation of an aquatic feed, wherein the aquatic feed comprises fishing bait, fish feed, shrimp feed, crab feed, turtle feed.

The beneficial effects of the present disclosure are as below.

1. The treatment agent solution prepared by the present disclosure may slowly absorbed into the intestinal tracts of the insects in a living state during the soaking of live insects. This gives enough time for the sediment in the intestinal tracts of the insects to be discharged, and the nitrate and/or nitrite can combine with the hemoglobin of insects to form nitrosyl hemoglobin, which has a bacteriostatic effect and can effectively kill the pathogens in the insects. Furthermore, under the action of antioxidants such as ascorbic acid or isoascorbic acid of the treatment agent solution prepared by the present disclosure, the nitrate ions and/or nitrite ions in nitrate and/or nitrite are reduced to nitric oxide, while trivalent iron in the hemoglobin of insects is reduced to divalent iron, and under the anoxic environment in insects, the hemoglobin combined with divalent iron forms nitrosoferric hemoglobin, which make the iron in the hemoglobin of insects more stable and less prone to browning. Compared with the natural red color, the insects after treatment has a purple-red color, which has a better effect of attracting aquatic animals.

2. The treatment method of the present disclosure can turn the hemoglobin in insects into stable, bright, purple-red nitrosoferric hemoglobin, and change the color of the insects to purple-red, which has a better attractiveness effect for aquaculture animals, improving its attractiveness and palatability for aquaculture animals.

3. The treatment method provided by the disclosure is low in cost and easy to operate, and the obtained insects or their larvae products have complete nutritional preservation, and are not easy to oxidize and spoil and cause off-flavors due to microbial growth. By changing the color and maintaining the original flavor to a greater extent, the insects are more easily detected and eaten by aquaculture animals such as fish, shrimp, crabs, turtles, etc., and have excellent attractiveness effects on aquaculture animals. The obtained insects or their larvae have a great potential in the preparation of aquatic feeds.

## BRIEF DESCRIPTION OF DRAWINGS

The present application is further described below in conjunction with the accompanying figures and examples, wherein:

Figure 1 is a schematic diagram of for a method for treating insects or their larvae as described in the present disclosure;
Figure 2 shows the semi-finished products made in Example 1 and Comparative Examples 1-3 of the present disclosure;
Figure 3 shows the frozen product made in Example 1 and Comparative Examples 1-3 of the present disclosure; and
Figure 4 shows the freeze-dried products made in Example 1 and Comparative Examples 1-3 of the present disclosure.

## DETAILED DESCRIPTION

The following will be a clear and complete description of the conception of the present disclosure and the technical effects produced in conjunction with the examples, in order to fully understand the purpose, features and effects of the present disclosure. Obviously, the described examples are only a part of the examples of the present disclosure, not all of them. Based on the examples of the present disclosure, other examples obtained by a person skilled in the art without creative labor fall within the scope of protection of the present disclosure.

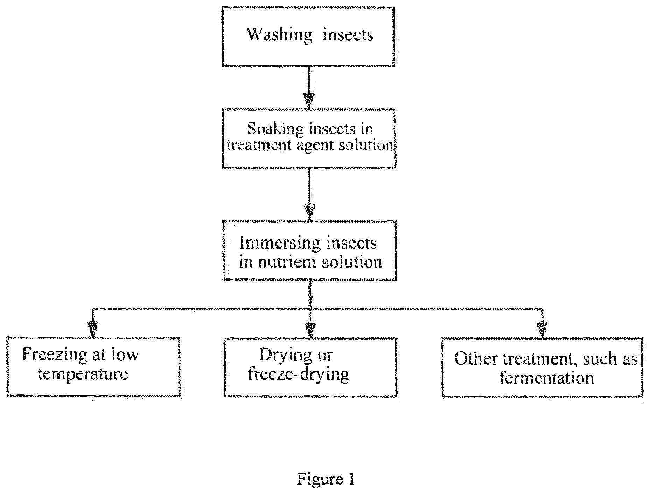

The present disclosure provides a method for treating insects or their larvae, and Figure 1 shows the main steps of the method for treating insects or their larvae described in the present disclosure.

In all examples of the present disclosure, the room temperature is 25°C.

In the examples of the present disclosure, the complex vitamins includes the following components by mass percentage: vitamin $B_1$ 0.3%, vitamin $B_2$ 0.25%, vitamin $B_3$ 0.7%, vitamin $B_{12}$ 0.08%, vitamin C 5%, vitamin D 2%, vitamin E 10%, and the remaining component is water; and the complex trace elements include the following components by mass percentage: calcium 2.5%, iodine 0.02%, zinc 1%, copper 0.15%, cobalt 0.002%, selenium 0.00001%, and the remaining component is water. The complex vitamins and complex trace elements are purchased from Guangdong Maritech Marine Biotechnology Co., Ltd.

The experimental materials and reagents used in the examples of the present disclosure, unless otherwise specified, are all routinely available commercially.

**Example 1**

This example provided a method for treating insects or their larvae, comprising:

(1) washing 100 kg of Chironomidae larvae (also known as blood worms, both blood worms and Chironomidae larvae mentioned in the present disclosure are the same species) with water to remove surface impurities;

(2) preparing 5 kg of treatment agent solution comprising the following components by mass percentage: potassium nitrate 5%, sodium nitrite 5%, ascorbic acid 20% and water 70%; placing the washed blood worms in the prepared treatment agent solution at room temperature for soaking for 12 hours; and then taking out the soaked blood worms;

(3) preparing 10 kg of nutrient solution comprising the following components by mass percentage: 10% of complex vitamins, 1% of complex trace elements and 89% of water; immersing the soaked blood worms in the prepared nutrient solution for 1.5 hours; and then taking out the immersed blood worms for draining water to obtain semi-finished products.

The semi-finished products were treated in the following different ways: (a) quick-frozen at a temperature of -30°C for 30 minutes and then packaged to obtain frozen products; (b) freeze-dried under a vacuum at a temperature of -25°C for 20 hours to obtain freeze-dried products.

**Example 2**

This example provided a method for treating insects or their larvae, comprising:

(1) washing 100 kg of earthworms with water to remove surface impurities:

(2) preparing 5 kg of treatment agent solution comprising the following components by mass percentage: sodium nitrite 15%, isoascorbic acid 25% and water 60%; placing the washed earthworms in the prepared treatment agent solution at room temperature for soaking for 12 hours; and then taking out the soaked earthworms;

(3) preparing 10 kg of nutrient solution comprising the following components by mass percentage: 5% of complex vitamins, 1% of complex trace elements, and 94% of water; immersing the soaked earthworms in the prepared nutrient solution for 0.5 hours; and taking out the immersed earthworms for draining water, quick-freeing and packaging.

**Example 3**

This example provided a method for treating insects or their larvae, comprising:

(1) washing 100 kg of water earthworms with water to remove surface impurities;

(2) preparing 5 kg of treatment agent solution comprising the following components by mass percentage: potassium nitrate 15%, sodium nitrite 5%, ascorbic acid 20% and water 60%; placing the washed water earthworms in the prepared treatment agent solution at room temperature for soaking for 16 hours; and then taking out the soaked water earthworms;

(3) preparing 10 kg of nutrient solution comprising the following components by mass percentage: 5% of complex vitamins, 3% of complex trace elements, and 92% of water; immersing the soaked water earthworms in the prepared nutrient solution for 2 hours; and taking out the immersed water earthworms for draining water, quick-freeing and packaging.

**Example 4**

This example provided a method for treating insects or their larvae, comprising:

(1) washing 100 kg of blood worms with water to remove surface impurities;

(2) preparing 5 kg of treatment agent solution comprising the following components by mass percentage: sodium nitrite 15%, ascorbic acid 20% and water 65%; placing the washed blood worms in the prepared treatment agent solution at room temperature for soaking for 16 hours; and then taking out the soaked blood worms;

(3) preparing 10 kg of nutrient solution comprising the following components by mass percentage: 12% of complex vitamins, 1% of complex trace elements and 87% of water; immersing the soaked blood worms in the prepared nutrient solution for 3 hours; and then taking out the immersed blood worms for draining water, quick-freeing, freeze-drying and packaging.

**Example 5**

This example provided a method for treating insects or their larvae, comprising:

(1) washing 100 kg of water earthworms with water to remove surface impurities;

(2) preparing 5 kg of treatment agent solution comprising the following components by mass percentage: potassium nitrite 4%, isoascorbic acid 40% and water 56%; placing the washed water earthworms in the prepared treatment agent solution at room temperature for soaking for 3 hours; and then taking out the soaked water earthworms;

(3) preparing 10 kg of nutrient solution comprising the following components by mass percentage: 8% of complex vitamins, 0.5% of complex trace elements and 91.5% of water; immersing the soaked water earthworms in the prepared nutrient solution for 0.5 hours; and then taking out the immersed water earthworms for draining water, quick-freeing, freeze-drying and packaging.

**Example 6**

This example provided a method for treating insects or their larvae, comprising:

(1) washing 100 kg of fly maggots with water to remove surface impurities;

(2) preparing 5 kg of treatment agent solution comprising the following components by mass percentage: sodium nitrate 20%, ascorbic acid 40% and water 40%; placing the washed fly maggots in the prepared treatment agent solution at room temperature for soaking for 0.5 hours; and then taking out the soaked fly maggots;

(3) preparing 10 kg of nutrient solution comprising the following components by mass percentage: 5% of complex vitamins, 2% of complex trace elements and 93% of water; immersing the soaked fly maggots in the prepared nutrient solution for 0.5 hours; and then taking out the immersed fly maggots for draining water, quick-freeing, freeze-drying and packaging.

**Example 7**

This example provided a method for treating insects or their larvae, comprising:

(1) washing 100 kg of blood worms with water to remove surface impurities;

(2) preparing 10 kg of the treatment agent solution comprising the following components by mass percentage: 20% potassium nitrite, 30% ascorbic acid and 50% water; placing the washed blood worms in the prepared treatment agent solution at room temperature for soaking for 15 hours; and then taking out the soaked blood worms:

(3) preparing 10 kg of nutrient solution comprising the following components by mass percentage: 8% of complex vitamins, 2% of complex trace elements and 90% of water; immersing the soaked blood worms in the prepared nutrient solution for 3 hours; taking out the immersed blood worms for draining water to obtain treated blood worms; homogenizing the treated blood worms with a grinder; and incubating the homogenized blood worms with *Lactobacillus plantarum* fermentation solution amounted 1% of the total mass of homogenized blood worms at room temperature for 5 days.

**Example 8**

This example provided a method for treating insects or their larvae, comprising:

(1) washing 100 kg of blood worms with water to remove surface impurities;

(2) preparing 10 kg of treatment agent solution comprising the following components by mass percentage: sodium nitrite 12%, tea polyphenols 10% and water 78%; placing the washed water earthworms in the prepared treatment agent solution at room temperature for soaking for 14 hours; and then taking out the soaked blood worms;

(3) preparing 10 kg of nutrient solution comprising the following components by mass percentage: 6% of complex vitamins, 1% of complex trace elements, and 93% of water; immersing the soaked blood worms in the prepared nutrient solution for 1 hours; and then taking out the immersed blood worms for draining water, quick-freeing and packaging.

**Example 9**

This example provided a method for treating insects or their larvae, comprising:

(1) washing 100 kg of blood worms with water to remove surface impurities;

(2) preparing 5 kg of treatment agent solution comprising the following components by mass percentage: sodium nitrite 8%, tea polyphenols 20% and water 72%; placing the washed blood worms in the prepared treatment agent solution at room temperature for soaking for 10 hours; and then taking out the soaked blood worms; and

(3) preparing 10 kg of nutrient solution comprising the following components by mass percentage: 15% of complex vitamins, 1% of complex trace elements and 84% of water; immersing the soaked blood worms in the prepared nutrient solution for 1 hours; and then taking out the immersed blood worms for draining water, quick-freeing, freeze-drying and packaging.

**Example 10**

This example provided a method for treating insects or their larvae, comprising:

(1) washing 100 kg of sandworms with water to remove surface impurities;

(2) preparing 5 kg of treatment agent solution comprising the following components by mass percentage: potassium nitrite 6%, nicotinamide 15% and water 79%; placing the washed sandworms in the prepared treatment agent solution at room temperature for soaking for 8 hours; and then taking out the soaked sandworms;

(3) preparing 10 kg of nutrient solution comprising the following components by mass percentage: 10% of complex vitamins, 2% of complex trace elements, and 88% of water; immersing the soaked sandworms in the prepared nutrient solution for 0.5 hours; and then taking out the immersed sandworms for draining water, quick-freeing, and packaging.

**Example 11**

This example provided a method for treating insects or their larvae, comprising:

(1) washing 100 kg of sandworms with water to remove surface impurities;

(2) preparing 10 kg of treatment agent solution comprising the following components by mass percentage: potassium nitrite 8%. nicotinamide 30% and water 62%; placing the washed sandworms in the prepared treatment agent solution at room temperature for soaking for 8 hours; and then taking out the soaked sandworms;

(3) preparing 10kg of nutrient solution comprising the following components by mass percentage: 10% of complex vitamins, 2% of complex trace elements, and 88% of water; immersing the soaked sandworms in the prepared nutrient solution for 0.5 hours; and then taking out the immersed sandworms for draining water, quick-freeing, and packaging.

**Example 12**

This example provided a method for treating insects or their larvae, comprising:

(1) washing 100 kg of sandworms with water to remove surface impurities;

(2) preparing 10 kg of treatment agent solution comprising the following components by mass percentage: potassium nitrite 10%, ascorbic acid 25% and water 65%; placing the washed sandworms in the prepared treatment agent solution at room temperature for soaking for 12 hours; and then taking out the soaked sandworms for quick-freezing, freeze-drying and packaging.

**Example 13**

This example provided a method for treating insects or their larvae, comprising:

(1) washing 100 kg of blood worms with water to remove surface impurities;

(2) preparing 10 kg of treatment agent solution comprising the following components by mass percentage: potassium nitrite 20%, ascorbic acid 20% and water 60%; placing the washed blood worms in the prepared treatment agent solution at room temperature for soaking for 16 hours; and then taking out the soaked blood worms for quick-freezing, freeze-drying and packaging.

**Comparative Example 1**

Comparative Example 1 provided a traditional treatment method of the current commercially available insects or their larvae, comprising:

(1) washing 100 kg of blood worms with water to remove surface impurities to obtain semi-finished products; and

(2) subjecting the semi-finished products to the following different post-treatments: (a) quick-freezing at -30°C for 30 minutes and packaging to obtain frozen products; (b) freeze-drying under a vacuum at a temperature of -25°C for 20 hours to obtain freeze-dried products.

**Comparative Example 2**

Comparative Example 2 provided a method for treating insects or their larvae, comprising:

(1) washing 100 kg of blood worms with water to remove surface impurities;

(2) preparing 5 kg of ascorbic acid solution comprising the following components by mass percentage: 20% ascorbic acid and 80% water; placing the washed blood worms in the prepared treatment agent solution at room temperature for soaking for 12 hours; and taking out the soaked blood worms; and

(3) draining the soaked blood worms to obtain semi-finished products.

The semi-finished products were treated in the following different ways: (a) quick-frozen at a temperature of -30°C for 30 minutes and then packaged to obtain frozen products; (b) freeze-dried under a vacuum at a temperature of -25°C for 20 hours to obtain freeze-dried products.

**Comparative Example 3**

Comparative Example 3 provided a method for treating insects or their larvae, comprising:

(1) washing 100 kg of blood worms with water to remove surface impurities;

(2) preparing 5 kg of nitrate solution comprising the following components by mass percentage: potassium nitrate 5%, sodium nitrite 5% and water 90%; placing the washed blood worms in the prepared treatment agent solution at room temperature for soaking for 12 hours; and then taking out the soaked blood worms;

(3) draining the soaked blood worms to obtain semi-finished products.

The semi-finished products were treated in the following different ways: (a) quick-frozen at a temperature of -30°C for 30 minutes and then packaged to obtain frozen products; (b) freeze-dried under a vacuum at a temperature of -25°C for 20 hours to obtain freeze-dried products.

**The effect comparison of Example 1 and Comparative Examples 1-3**

The effects of the insect samples obtained by the treatment method of Example 1 of the present disclosure were compared with those of insect samples obtained by the treatment methods of Comparative Examples 1-3 in terms of appearance and color of semi-finished and finished products, the synthesis effect of nitrosohemoglobin, bacterial inhibition and preservation resistance, attractiveness when used as bait for fishing, and attractiveness when used as aquatic feeds.

1. Appearance and color evaluation

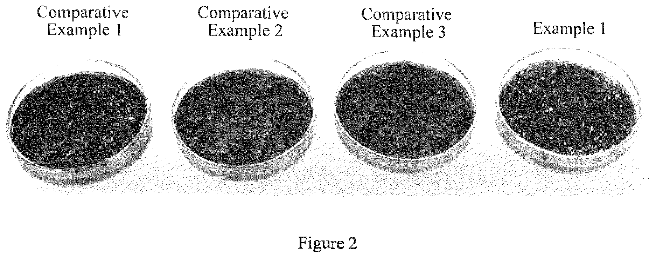

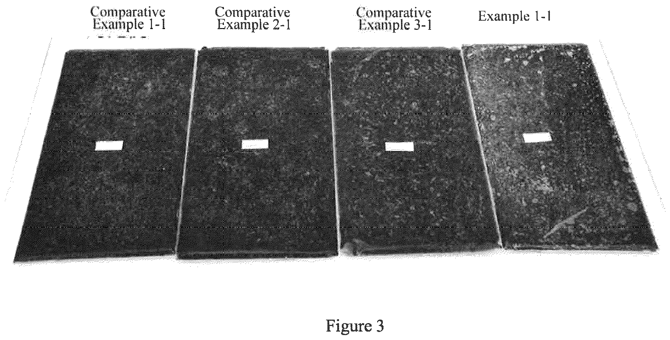

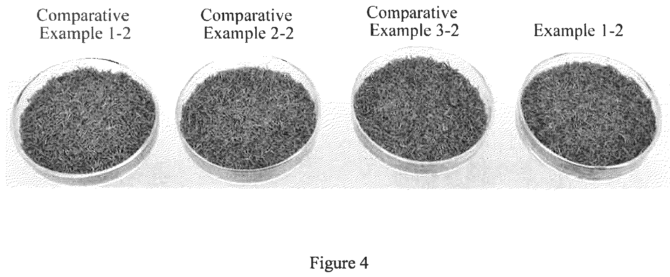

The semi-finished products, frozen products and freeze-dried products obtained in Example 1, Comparative Example 1, Comparative Example 2 and Comparative Example 3 were compared with the naked eye under the same observation field and recorded by taking photographs (as shown in Figures 2-4). The frozen products were marked as Example 1-1, Comparative Example 1-1, Comparative Example 2-1 and Comparative Example 3-1, and the freeze-dried products were marked as Example 1-2, Comparative Example 1-2, Comparative Example 2-2 and Comparative Example 3-2, respectively.
From Figures. 2-4, it can be found that the blood worm products made in Example 1 are more stable and brighter in color, and have better appearances compared to the blood worm products made in Comparative Examples 1-3.

2. Evaluation of the synthesis effect of nitrosohemoglobin

(1) Test of the nitrosohemoglobin content of product

Determination by acetone method: 25 g of semi-finished products, frozen products and freeze-dried products obtained in Example 1 and Comparative Examples 1-3 were weighed as test samples, and placed in a sterile homogenizing cup containing 225 ml of normal saline, and then homogenized in a mixer at 8000 rpm for 2 min. The homogenized sample solution was filtered, and the filtrate was freeze-dried and transferred to a stoppered test tube, dissolved with 80% acetone solution by volume, and left for a certain time in the dark at room temperature until the nitrohemochromogen was completely extracted. The filtrate was taken as test sample, and measured the absorbance of the test sample at 540 nm by using a UV spectrophotometer with acetone solution at 80% mass concentration as a blank control, and the results were shown in Table 1.

(2) Test results

Table 1 Contents of nitrosohemoglobin of the samples absorbance value at 540 nm)

| Test sample | Example 1 semi-finished product | Comparative Example 1 semi-finished product | Comparative Example 2 semi-finished product | Comparative Example 3 semi-finished product |
|---|---|---|---|---|
| Absorbance value | 0.82 | 0.64 | 0.71 | 0.74 |
| Test sample | Example 1-1 frozen product | Comparative Example 1-1 frozen product | Comparative Example 2-1 frozen product | Comparative Example 3-1 frozen product |
| Absorbance value | 0.78 | 0.67 | 0.71 | 0.72 |

(continued)

| Test sample | Example 1-2 freeze-dried product | Comparative Example 1-2 freeze-dried product | Comparative Example 2-2 freeze-dried product | Comparative Example 3-2 freeze-dried product |
|---|---|---|---|---|
| Absorbance value | 0.79 | 0.60 | 0.67 | 0.68 |

The results show that the treatment method of the present disclosure can obtain insects and their larvae products containing high concentrations of nitrosohemoglobin, which can give the insects a bright purple-red color, improve the appearance of the products and achieve better attractiveness effects.

3. Bacterial inhibition and preservation resistance evaluation

(1) Test sample preparation

50 grams of the semi-finished products obtained in Example 1, Comparative Examples 1-3 were randomly taken and placed at room temperature for 6 hours, 12 hours, 1 day, 3 days, 5 days and 7 days, respectively to evaluate the bacterial inhibition ability by total bacteria count test. Another 50 grams samples were randomly taken for the volatile salt nitrogen test and the nitrosohemoglobin content test performed for 3, 6, 12, 18, 24 and 36 months under specific storage conditions (-20°C for semi-finished and frozen products, and room temperature for freeze-dried products).

(2) Total bacteria count test

25 g of the above test samples stored at room temperature were weighed, and placed in a sterile homogenizing cup containing 225 ml of normal saline, and then homogenized in a mixer at 8000 rpm for 2 min. After incremental gradient dilution at 10 times, 1 mL of homogenate sample was spread in a sterile dish containing a plate counting agar medium, applied evenly, and incubated at a temperature of 37°C $\pm$ 1°C for 48 h, wherein the plate counting agar medium was prepared by a method comprising weighing tryptone 5.0 g, yeast extract 2.5 g, glucose 1.0 g and agar 15.0 g, adding distilled water 1000 mL, heating to dissolve, adjusting pH to 7.0 $\pm$ 0.2, and autoclaving at 121 °C for 15 minutes. Colony plate counts were performed, and the results were shown in Table 2.

(3) Volatile salt nitrogen test

The test samples preserved under the above specific storage conditions were crushed, mixed thoroughly and then packed into a mill-mouth flask. 10 g of the sample was weighed into a 250 ml conical flask with a stopper, 100 ml of distilled water was added and shaken well for 30 minutes. 20 ml of boric acid solution with 2% mass concentration was added in 150 ml conical flask, added 2 drops of mixed indicator prepared by mixing a methyl red solution with 0.1% mass concentration in ethanol and an bromocresol green solution with 0.5% mass concentration in ethanol in equal volume, the end of the condenser tube of the semi-micro distillation device was submerged into this solution, and then 2 drops of methyl red indicator and 0.01 mol/L sulfuric acid solution were added to the water of the steam generator of the distillation unit to keep the solution orange-red. 10 ml of sample solution was accurately pipetted into the reaction chamber of the distillation device, the inlet was flushed with a small amount of distilled water, and then 10 ml of magnesium oxide suspension with a mass concentration of 1.0% was added. Water was added to seal the inlet to prevent air leakage, distillation was conducted for 4 minutes so that the end of the condenser tube left the surface of the absorption solution, and then distillation was conducted for 1 minute again, the end of the condenser tube was washed with distilled water, and the washing solution all flowed into the absorption solution. After absorption of ammonia, the absorption solution was titrated with 0.01 mol/L of hydrochloric acid standard solution immediately, until the solution changed from blue-green to gray-red. The blank reagent determination was conducted, and the results were shown in Table 3. In addition, 25g of corresponding samples were weighed separately, and the absorbance of the test samples under different storage time was determined by the acetone method with reference to the nitrosohemoglobin content testing procedure in this Example, and the results were shown in Table 4.

The content of volatile salt nitrogen $\omega$ (mg/g) of the test sample was calculated by the following equation.

$$\omega = \frac{(V1 - V2) \times C \times 14}{m \times \frac{10}{100}} \times 100$$

In the above equation, V1 represents the volume of hydrochloric acid standard solution consumed by the sample solution for the determination, in mL;

V2 represents the volume of hydrochloric acid standard solution consumed by the blank reagent, in mL;

C represents the actual concentration of the standard solution of hydrochloric acid, in mol/L; and

m represents the mass of sample, in gram.

(4) Test results

Table 2 Total bacterial counts (log cfu/g) of the test samples obtained in Examples

| Test sample storage time | 6 hours | 12 hours | 1 day | 3 days | 5 days | 7 days |
|---|---|---|---|---|---|---|
| Example 1 semi-finished product | 3.54 | 3.45 | 3.94 | 3.51 | 2.79 | 2.41 |
| Comparative Example 1 semi-finished product | 8.46 | 9.72 | 11.08 | 12.40 | 12.86 | 12.77 |
| Comparative Example 2 semi-finished product | 6.76 | 7.46 | 8.82 | 9.80 | 11.18 | 11.92 |
| Comparative Example 3 semi-finished product | 5.89 | 5.98 | 5.41 | 6.68 | 5.94 | 4.40 |

Table 3 Volatile salt nitrogen content (mg/g) of the test samples obtained in Examples

| Test sample storage time | 3 months | 6 months | 12 months | 18 months | 24 months | 36 months |
|---|---|---|---|---|---|---|
| Example 1 semi-finished product | 9.38 | 12.11 | 14.27 | 16.96 | 20.31 | 23.55 |
| Comparative Example 1 semi-finished product | 18.27 | 23.47 | 26.19 | 30.26 | 35.44 | 43.72 |
| Comparative Example 2 semi-finished product | 13.28 | 16.86 | 20.06 | 24.11 | 28.73 | 31.24 |
| Comparative Example 3 semi-finished product | 12.17 | 17.35 | 23.19 | 25.76 | 26.55 | 29.54 |
| Example 1-1 frozen product | 8.58 | 11.27 | 13.92 | 16.28 | 19.35 | 22.25 |
| Comparative Example 1-1 frozen product | 16.05 | 19.13 | 22.26 | 25.16 | 27.71 | 35.13 |
| Comparative Example 2-1 frozen product | 13.53 | 16.72 | 19.06 | 21.56 | 24.62 | 30.82 |

(continued)

| Test sample storage time | 3 months | 6 months | 12 months | 18 months | 24 months | 36 months |
|---|---|---|---|---|---|---|
| Comparative Example 3-1 frozen product | 10.77 | 13.24 | 18.39 | 19.33 | 23.07 | 25.35 |
| Example 1-2 freeze-dried product | 6.42 | 9.20 | 11.95 | 13.88 | 15.74 | 18.35 |
| Comparative Example 1-2 freeze-dried product | 15.75 | 17.25 | 21.71 | 26.55 | 32.43 | 39.16 |
| Comparative Example 2-2 freeze-dried product | 11.66 | 14.39 | 18.56 | 23.43 | 27.62 | 33.85 |
| Comparative Example 3-2 freeze-dried product | 9.91 | 13.07 | 16.88 | 19.19 | 23.58 | 28.74 |

Table 4 Content of nitrosohemoglobin after storage of the test samples obtained in Examples (absorbance value at 540 nm)

| Test sample storage time | 3 months | 6 months | 12 months | 18 months | 24 months | 36 months |
|---|---|---|---|---|---|---|
| Example 1 semi-finished product | 0.82 | 0.80 | 0.77 | 0.75 | 0.72 | 0.70 |
| Comparative Example 1 semi-finished product | 0.55 | 0.52 | 0.50 | 0.45 | 0.42 | 0.37 |
| Comparative Example 2 semi-finished product | 0.70 | 0.70 | 0.66 | 0.60 | 0.52 | 0.47 |
| Comparative Example 3 semi-finished product | 0.73 | 0.71 | 0.70 | 0.66 | 0.63 | 0.61 |
| Example 1-1 frozen product | 0.78 | 0.77 | 0.74 | 0.70 | 0.69 | 0.67 |
| Comparative Example 1-1 frozen product | 0.60 | 0.56 | 0.53 | 0.50 | 0.48 | 0.47 |
| Comparative Example 2-1 frozen product | 0.70 | 0.68 | 0.65 | 0.62 | 0.58 | 0.51 |
| Comparative Example 3-1 frozen product | 0.72 | 0.69 | 0.69 | 0.65 | 0.61 | 0.56 |
| Example 1-2 freeze-dried product | 0.79 | 0.79 | 0.78 | 0.75 | 0.73 | 0.71 |

(continued)

| Test sample storage time | 3 months | 6 months | 12 months | 18 months | 24 months | 36 months |
|---|---|---|---|---|---|---|
| Comparative Example 1-2 freeze-dried product | 0.52 | 0.50 | 0.47 | 0.45 | 0.45 | 0.42 |
| Comparative Example 2-2 freeze-dried product | 0.65 | 0.63 | 0.56 | 0.53 | 0.50 | 0.48 |
| Comparative Example 3-2 freeze-dried product | 0.68 | 0.66 | 0.60 | 0.58 | 0.55 | 0.51 |

The results show that the treatment agent and the method as described in the present disclosure have advantages of good bacteriostatic effect and durable storage. It can be found from Table 2 that the blood worm products obtained in the Examples can effectively inhibit the growth of bacteria under the same storage conditions. The volatile salt nitrogen in Table 3 refers to the alkaline nitrogenous substances such as ammonia and amines produced by the decomposition of proteins in the spoilage process of animal food due to the action of enzymes and bacteria:. The alkaline nitrogenous substances are volatile, and the higher content of the alkaline nitrogenous substances indicates that the amino acids are more destroyed, especially methionine and tyrosine, so the nutritional value is greatly affected. It can be seen from Table 3 that the volatile salt nitrogen content of the blood worm products of Example 1 is lower than those of Comparative Examples 1-3. It can be seen from Table 4 that the nitrosohemoglobin content of the untreated samples of Comparative Example 1 decreased significantly after 3 months of storage, the nitrosohemoglobin content of the samples of Comparative Examples 2 and 3 also began to decrease significantly after 12 months of storage, while the samples of Example 1 could maintain a higher level of nitrosohemoglobin until 36 months of storage. The test results show that the treatment agent and the method as described in the present disclosure can effectively inhibit the growth of bacteria and keep the proteins in the insects or their larvae from being decomposed, and can also better maintain the nitrosohemoglobin content in the insects under long storage, ensuring the quality and appearance of the insects.

3. Evaluation of the attractiveness of treated insects used as bait for fishing

(1) Fishes used in experiment and feeding management

30 cichlids with an average weight of 87.3 $\pm$ 4.7 g were kept in an indoor glass aquarium (120 cm length $\times$ 30 cm width $\times$ 70 cm height) and fed a basal diet at a daily feeding rate of 2% of the fish weight, and the cichlids were tested after 15 days of feeding. The aquarium was equipped with water circulation filter and heating device, and had a ffective water volume of 200 liters. Fully aerated tap water was used as the water source during the test period, and the water temperature was controlled at 21 $\pm$ 1°C with continuous oxygenation, and the water was changed once a day, about 1/3 of the water was changed each time.

(2) Bait preparation

10 semi-finished products blood worms of Example 1 (Example 1-1), 10 frozen products blood worms (Example 1-2) and 10 freeze-dried products blood worms (Example 1-3) were taken respectively as fishing baits, and marked as test group. 10 semi-finished products blood worms (Comparative Example 1-1, Comparative Example 2-1, Comparative Example 3-1) , 10 frozen products blood worms (Comparative Example 1-2, Comparative Example 2-2, Comparative Example 3-2) and freeze-dried products (Comparative Example 1-3, Comparative Example 2-3, Comparative Example 3-3) of Comparative Example 1, Comparative Example 2, and Comparative Example 3 were taken as fishing baits, and marked as Control Group 1, Control Group 2, and Control Group 3. A total of 12 groups of baits were tied with cotton thread and distributed equally at 10 cm intervals in a 120 cm long aquarium, placed at the middle of the width, with the bait located at a depth of 25 cm below the water surface.

(3) Evaluation method of attractiveness effect

The number of pecking bites of the fishes on 12 groups of baits within 15 minutes was observed and recorded separately. The experiment was conducted at each interval of one day and repeated five times, and the data were expressed as the mean of five repetitions ± standard deviation. The number of pecking bites was used to judge the attractiveness effect of the baits on cichlids, and the results were shown in Table 5.

The semi-finished products, frozen products and freeze-dried products of Example 1, and the semi-finished products, frozen products and freeze-dried products of Comparative Example 1 were used as samples. One cichlid was separated by a transparent baffle on one side of the aquarium to observe and record its preferential feeding samples, and 30 cichlids in each group were used for the determination of the number of pecking bites., The experiment was conducted at each interval of one day and repeated five times, and the data were expressed as the mean of five repetitions ± standard deviation. The results were shown in Table 6.

(4) Test results

Table 5 Evaluation of the attractiveness of the experimental samples as fishing baits

| Test sample | Example 1 semi-finished product | Comparative Example 1 semi-finished product | Comparative Example 2 semi-finished product | Comparative Example 3 semi-finished product |
|---|---|---|---|---|
| Number of pecking bites | 68±7 | 34±6 | 48±6 | 53±5 |
| Test sample | Example 1-1 frozen product | Comparative Example 1-1 frozen product | Comparative Example 2-1 frozen product | Comparative Example 3-1 frozen product |
| Number of pecking bites | 58±4 | 25±5 | 44±7 | 45±9 |
| Test sample | Example 1-2 freeze-dried product | Comparative Example 1-2 freeze-dried product | Comparative Example 2-2 freeze-dried product | Comparative Example 3-2 freeze-dried product |
| Number of pecking bites | 53±9 | 39±5 | 41±3 | 40±4 |

Table 6 Preferential feeding evaluation of test samples as fishing baits

| Test sample | Example 1 semi-finished product | Comparative Example 1 semi-finished product |
|---|---|---|
| Number of preferential feeding | 27.8±0.7 | 2.2±0.7 |
| Test sample | Example 1-1 frozen product | Comparative Example 1-1 frozen product |
| Number of preferential feeding | 28.2±0.7 | 1.8±0.7 |
| Test sample | Example 1-2 freeze-dried product | Comparative Example 1-2 freeze-dried product |
| Number of preferential feeding | 28.0±0.9 | 2.0±0.9 |

The results show that the blood worms prepared in Example 1 have good attractiveness when used as baits, and the number of pecking bites of cichlids on the blood worms made in Example 1 was higher than that of the blood worms made in Comparative Examples 1-3 in the test. Compared with Comparative Example 1, the blood worms produced in Example 1 have a higher number of preferential feeding, and cichlids have a high probability of preferential eating the blood worm products of Example 1, which indicates that the samples are brightly colored and more attractive to fishes. The products produced by this treatment method are not only durable to storage, but also have good attractiveness and have broad application prospects in fish baits.

4. Evaluation of the attractiveness of treated insects as aquatic feeds

(1) Fishes used in experiment and feeding management

Fishes used in experiment and feeding management may be referred to the section of "fishes used in experiment and feeding management" as described in the above baits attractiveness evaluation.

(2) Evaluation of attractiveness

Using the free feeding method, with reference to the grouping in the baits attractiveness evaluation described above, the preparation of the baits added was adjusted to directly add the corresponding blood worms as feeds for feeding, which means each group was fed with a corresponding excess of 100 g of feed, respectively. The attractiveness was expressed as the mean value of the attractiveness index calculated by observation at 30 min (when there was a surplus of feeds). The experiment was conducted at each interval of one day, and repeated five times. The data were expressed as the mean of five repetitions $\pm$ standard deviation, and the results were shown in Table 7.

The formula for calculating the attractiveness index is (in the case of freeze-dried products, the residual feed needs to be dried before weighing)

$$\text{Attractiveness index} = 1 - \frac{\text{Remaining feeds weight (g)}}{\text{added feed weight (g)}}$$

(3) Test result

Table 7 Evaluation of theattractiveness of the test samples as aquatic feeds

| Test sample | Example 1 semi-finished product | Comparative Example 1 semi-finished product | Comparative Example 2 semi-finished product | Comparative Example 3 semi-finished product |
|---|---|---|---|---|
| Attractiveness index | 0.95±0.02 | 0.68±0.13 | 0.73±0.06 | 0.81±0.11 |
| Test sample | Example 1 -1 frozen product | Comparative Example 1-1 frozen product | Comparative Example 2-1 frozen product | Comparative Example 3-1 frozen product |
| Attractiveness index | 0.90±0.02 | 0.71±0.09 | 0.68±0.10 | 0.84±0.02 |
| Test sample | Example 1-2 freeze-dried product | Comparative Example 1-2 freeze-dried product | Comparative Example 2-2 freeze-dried product | Comparative Example 3-2 freeze-dried product |
| Attractiveness index | 0.93±0.03 | 0.66±0.02 | 0.69±0.04 | 0.74±0.14 |

The results show that the feeding rate of the blood worms prepared in Example 1 was significantly higher than that of the other Comparative Examples, and the attractiveness index was higher than that of the blood worms prepared in Comparative Examples 1-3, indicating that the products made by this treatment methods are not only durable, but also have good palatability and attractiveness, which have a wide application prospect in aquatic feeds.

The above experimental results show that the insect products treated with this technical method have the advantages of bright color, anti-bacteria and durable to storage, high attractiveness and high palatability, which have great potential for applications in aquatic feeds.

The Examples of the present disclosure are described in detail above in conjunction with the accompanying drawings, but the present disclosure is not limited to the above Examples, and various variations may be made within the scope of knowledge possessed by a person of ordinary skill in the art to which they belong without departing from the purpose of the present disclosure. In addition, the Examples of the present disclosure and the features in the Examples can be combined with each other without conflict.

**Claims**

1. A treatment agent of insects or their larvae, comprising at least one selected from the group consisting of nitrate and nitrite, and at least one selected from the group consisting of ascorbic acid, isoascorbic acid, tea polyphenols and nicotinamide.

2. The treatment agent according to claim 1, wherein the treatment agent comprises 4 to 20% by weight of nitrate and/or 4 to 20% by weight of nitrite, and 10 to 40% by weight of ascorbic acid, isoascorbic acid, tea polyphenols or nicotinamide.

3. The treatment agent according to claim 1 or 2, wherein the nitrates is at least one selected from the group consisting of sodium nitrate, and potassium nitrate.

4. The treatment agent according to claim 1 or 2, wherein the nitrite is at least one selected from the group consisting of sodium nitrite, and potassium nitrite.

5. The treatment agent according to any one of claims 1-4, wherein the insects or their larvae are insects or their larvae containing hemoglobin.

6. The treatment agent according to claim 5, wherein the insects or their larvae containing hemoglobin comprise Chironomidae larvae, earthworms, water earthworms, fly maggots, and sandworms.

7. A method of treating insects or their larvae, comprising the steps of:

   S1. washing the insects or their larvae to obtain washed insects or their larvae; and
   S2. soaking the washed insects or their larvae in a solution of the treatment agent of any one of claims 1-6 to obtain soaked insects or their larvae; and taking the soaked insects or their larvae out for draining water to obtain treated insects or their larvae.

8. The method according to claim 7, wherein in Step S2, soaking the washed insects or their larvae in the solution of treatment agent for 0.5 to 16 hours.

9. The method according to claim 7 or 8, further comprising: after soaking in the solution of treatment agent, immersing the insects or their larvae in a nutrient solution for 0.5 to 3 hours.

10. The method according to claim 9, wherein the nutrient solution comprises from 5 to 15% by weight of complex vitamins, and from 0.5 to 3% by weight of complex trace elements.

11. The method according to claim 10, wherein the complex vitamins comprises one or more of vitamin $B_1$, vitamin $B_2$, vitamin $B_3$, Vitamin $B_{12}$, vitamin C, vitamin D, and vitamin E.

12. The method according to claim 10, wherein the complex trace elements comprises one or more of calcium, magnesium, phosphorus, zinc, manganese, copper, iron, and selenium.

13. An aquatic feed, comprising the insects or their larvae obtained by the method of any one of claims 7-12.

14. The aquatic feed according to claim 13, wherein the aquatic feed is at least one selected from the group consisting of fishing bait, fish feed, shrimp feed, turtle feed and crab feed.

15. Use of the insects or their larvae obtained by the method of any one of claims 7-12 in the preparation of an aquatic feed, wherein the aquatic feed is at least one selected from the group consisting of fishing bait, fish feed, shrimp feed, turtle feed and crab feed.

Figure 1

Comparative Example 1    Comparative Example 2    Comparative Example 3    Example 1

Figure 2

Figure 3

Comparative Example 1-2   Comparative Example 2-2   Comparative Example 3-2   Example 1-2

Figure 4

Europäisches
Patentamt

European
Patent Office

Office européen
des brevets

# EUROPEAN SEARCH REPORT

**Application Number**

EP 23 18 8907

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 679 002 A1 (NEUDORFF W GMBH KG [DE]) 12 July 2006 (2006-07-12) * paragraph [0007]; claims 1, 7, 8, 13, 14 * | 1 | INV. A23K10/20 A01K61/00 A23K10/30 A23K20/174 |
| X | CN 107 114 607 A (GUANGXI AGRICULTURAL VOCATIONAL COLLEGE) 1 September 2017 (2017-09-01) * claim 1; example 1 * | 13-15 | A23K20/20 A23K50/80 |
| X | US 2022/256892 A1 (FECHER YAIR M [IL]) 18 August 2022 (2022-08-18) * paragraph [0001]; claim 29; example 4 * * paragraph [0014] – paragraph [0026] * | 13-15 8-12 | |
| X | WO 2021/012300 A1 (INTERNATIONAL HEAVY INTELLIGENT COMPUTING CO LTD [CN]) 28 January 2021 (2021-01-28) * example 1 * | 1,7 8-12 2-6 | |
| Y A | | | |
| X | CN 109 007 281 A (SUZHOU JINGRUI ENVIRONMENTAL PROTECTION TECH CO LTD) 18 December 2018 (2018-12-18) * paragraphs [0026], [0031], [0048], [0063] * | 13-15 1-12 | TECHNICAL FIELDS SEARCHED (IPC) A23K A01K |
| A | | | |
| X | CN 1 159 968 C (HOU WENBIN [CN]) 4 August 2004 (2004-08-04) * paragraph [0001] – paragraph [0003] * * paragraphs [0006], [0017] * | 13-15 1-12 | |
| A | | | |
| A | CN 103 549 484 A (FENG CAIPING) 5 February 2014 (2014-02-05) * the whole document * | 1-15 | |

-/--

The present search report has been drawn up for all claims

| Place of search | Date of completion of the search | Examiner |
|---|---|---|
| Munich | 12 January 2024 | Steiner Ribeiro G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 23 18 8907

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | M. HENRY ET AL: "Review on the use of insects in the diet of farmed fish: Past and future", ANIMAL FEED SCIENCE AND TECHNOLOGY, vol. 203, 1 May 2015 (2015-05-01), pages 1-22, XP055263356, AMSTERDAM, NL ISSN: 0377-8401, DOI: 10.1016/j.anifeedsci.2015.03.001 * the whole document * | 1-15 | |
| A | WO 2020/199530 A1 (HUNAN HAIKUN AGRICULTURAL SCIENCE AND TECH CO LTD [CN]) 8 October 2020 (2020-10-08) * the whole document * | 1-15 | |

TECHNICAL FIELDS
SEARCHED      (IPC)

The present search report has been drawn up for all claims

| Place of search | Date of completion of the search | Examiner |
|---|---|---|
| Munich | 12 January 2024 | Steiner Ribeiro G. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
   ................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 23 18 8907

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2024

| Patent document cited in search report | | Publication date | Patent family member(s) | | Publication date |
|---|---|---|---|---|---|
| EP 1679002 | A1 | 12-07-2006 | AT | E381261 T1 | 15-01-2008 |
| | | | AT | E468013 T1 | 15-06-2010 |
| | | | DE | 60318259 T2 | 04-12-2008 |
| | | | DK | 1402776 T3 | 20-09-2010 |
| | | | DK | 1679002 T3 | 04-02-2008 |
| | | | EP | 1402776 A1 | 31-03-2004 |
| | | | EP | 1679002 A1 | 12-07-2006 |
| | | | ES | 2296238 T3 | 16-04-2008 |
| | | | ES | 2344634 T3 | 02-09-2010 |
| | | | PT | 1679002 E | 28-02-2008 |
| | | | US | 2004062785 A1 | 01-04-2004 |
| | | | US | 2007134284 A1 | 14-06-2007 |
| | | | US | 2011081425 A1 | 07-04-2011 |
| CN 107114607 | A | 01-09-2017 | NONE | | |
| US 2022256892 | A1 | 18-08-2022 | AU | 2020319179 A1 | 10-03-2022 |
| | | | CA | 3151371 A1 | 28-01-2021 |
| | | | CL | 2022000150 A1 | 09-09-2022 |
| | | | CN | 114423299 A | 29-04-2022 |
| | | | EP | 4003053 A1 | 01-06-2022 |
| | | | IL | 290017 A | 01-03-2022 |
| | | | KR | 20220044967 A | 12-04-2022 |
| | | | US | 2022256892 A1 | 18-08-2022 |
| | | | WO | 2021014443 A1 | 28-01-2021 |
| WO 2021012300 | A1 | 28-01-2021 | CN | 110250328 A | 20-09-2019 |
| | | | WO | 2021012300 A1 | 28-01-2021 |
| CN 109007281 | A | 18-12-2018 | NONE | | |
| CN 1159968 | C | 04-08-2004 | NONE | | |
| CN 103549484 | A | 05-02-2014 | NONE | | |
| WO 2020199530 | A1 | 08-10-2020 | CN | 109965154 A | 05-07-2019 |
| | | | WO | 2020199530 A1 | 08-10-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 109965154 A
- CN 109691615 A
- CN 113273639 A